# EUROPEAN PATENT SPECIFICATION

(11) **EP 0 547 665 B1**
(45) Date of publication and mention of the grant of the patent: **29.03.1995**
(21) Application number: 92203787.4
(22) Date of filing: 07.12.1992
(51) Int. Cl.: A61K 7/16

(54) **Oral hygiene products**
Mittel zur orale Verwendung
Produits à usage buccal

(30) Priority: 09.12.1991 GB 9126101
(43) Date of publication of application: 23.06.1993
(73) Proprietor: UNILEVER N.V., 3000 DK Rotterdam (NL); UNILEVER PLC, London EC4P 4BQ (GB)
(72) Inventor: Duckworth, Ralph Marsland, Unilever Research, Bebington, Wirral, Merseyside L63 3JW (GB); Robb, Ian Donald, Unilever Research, Bebington, Wirral, Merseyside L63 3JW (GB); Haley, Judith Anne, Unilever Research, Bebington, Wirral, Merseyside L63 3JW (GB); Jones, Christopher, Clarckson, Unilever Research, Bebington, Wirral, Merseyside L63 3JW (GB)
(74) Representative: van Gent, Jan Paulus

(56) References cited:
- EP-A- 0 228 209
- WO-A-91/08283
- FR-A- 2 647 012
- GB-A- 1 290 627

## Description

The present invention relates to oral hygiene products which contain a particulate therapeutic agent which is suitable for the treatment, prevention or reduction of dental disorders.

More particularly, the present invention relates to oral hygiene products in the form of liquid mouthwash compositions which comprise a particulate therapeutic agent which is stably suspended in the liquid mouthwash compositions.

In US-Patent Specification 4,837,007 (Duckworth, et al.) oral hygiene products have been disclosed which comprise a first and a second composition for admixing in the mouth or for admixing prior to introduction into the mouth. The first composition comprises an aqueous suspension of particles which slowly release a therapeutic agent in saliva, and the second composition comprises an aqueous solution of a cationic polymer. Saliva severely interferes with the deposition of particles onto the oral surfaces, and by bringing the particles into contact with the solution of the cationic polymer prior to or simultaneously with their introduction into the mouth the inhibiting effect of the saliva can be overcome and thereby an efficacious deposition of particles capable of releasing a therapeutic agent effected.

Although this US Patent Specification also mentions the possibility of a mouthwash composition comprising an aqueous suspension of the particles, said aqueous suspension containing dissolved therein the cationic polymer, no further information is provided about such compositions, the whole teaching of this US Patent Specification being directed solely to the two-compositions' system. Although these two-compositions' systems provide for an efficacious oral hygiene product, the use thereof may be somewhat inconvenient to the consumer in that the consumer has to mix the two compositions just prior to introduction into the mouth, or to treat the mouth with the two compositions sequentially. It would therefore be more convenient if these systems could be formulated into one single system, without losing its overall efficacy.

The cationic polymers, specifically exemplified in the above US-patent, e.g. Floc Aid and Reten, which have a molecular weight of 2,000,000 or higher, were found not to give a stable single system, and Jaguar (molecular weight 220,000) was found to give only a marginally stable single system.

It has now been found that such a single system can be obtained by stably suspending the particulate therapeutic agent in a liquid medium which contains specific polymers, which are selected from the group consisting of proteinaceous polymers, polysaccharidic polymers, copolymers of vinylpyrolidone with an acrylic monomer, and mixtures thereof, said polymers having an average molecular weight of 1,000,000 or less.

The proteinaceous polymers of the present invention should be capable of having a net positive charge under the pH conditions of use of the mouthwash composition. This does not necessarily mean the actual pH of the mouthwash composition itself, but rather the pH of oral fluid when the mouth is rinsed with the mouthwash composition. Consequently, the proteinaceous polymer is a cationic polymer under the pH conditions of use of the mouthwash composition. In general, the pH condition of use is 8 or less, and usually the mouthwash composition is formulated to have a pH of 8 or less, customarily in the order of about 5.5 to about 8.

Typical examples of suitable proteinaceous polymers are, gelatin, albumin, clupeine, and lysozyme; gelatin being the preferred proteinaceous polymer. A suitable gelatin is type A gelatin with a Bloom strength of 100.

The proteinaceous polymer is used in an amount of 0.0001-1%, preferably 0.001-0.75% by weight of the composition.

The polysaccharidic polymer of the present invention should also have a net positive charge at the pH of use of the mouthwash composition. Therefore, it should be a cationic polysaccharidic polymer, and typical examples thereof are quaternized cellulose derivatives as described in US Patent 3,472,840, available under the trade name Polymer JR, e.g. Polymer JR 100 and Polymer JR 400. Other suitable cationic cellulosic polymers are cationic starches, and quaternized cellulose/dimethyldialkylammonium chloride polymers available under the trade name Celquat. Another suitable polysaccharide polymer is chitosan. Where the polysaccharidic polymer is derived from a guar gum, it should have a cationic density as hereinafter defined of at least 0.0009.

The cationic polysaccharidic polymer is used in an amount of 0.0001-1%, preferably 0.001-0.5% by weight of the composition.

The copolymers of vinylpyrrolidone with an acrylic monomer should equally have a net positive charge at the pH of use of the mouthwash composition and should therefore be cationic. The acrylic monomer can be an acrylate or methacrylate or derivatives thereof, such as dimethylaminoethyl(meth)acrylate.

Suitable examples of such copolymers are copolymers of vinylpyrrolidone with dimethylaminoethylmethacrylate such as Gafquat 734 and 755 ex GAF.

The above copolymers are used in an amount of 0.0001-1%, preferably 0.001-0.5% by weight of the composition.

All the above types of polymers except those derived from a guar gum should have, under the conditions of use of the mouthwash composition, a cationic charge density of at least 0.0002. The polymers derived from a guar gum should have, as said above, a cationic charge density of at least 0.0009. The cationic charge density is the ratio of the number of charges on a polymer chain to the molecular weight of the polymer chain. The polymers should have a molecular weight of 1,000,000 Daltons or less.

Typical examples of sparingly-soluble, particulate therapeutic agents are sparingly-soluble fluorides like magnesium fluoride, calcium fluoride, strontium fluoride neighborite (NaMgF₃), sparingly-soluble zinc compounds like zinc citrate, zinc oxide, hydrotalcites, sparingly-soluble stannous salts like stannous pyrophosphate, and so on. Other particulate therapeutic agents are polymeric latices which release fluoride.

In general, the particle diameter of the particulate therapeutic agent should lie within the range of 0.01-5 »m, preferably 0.05-0.5 »m, as measured by transmission electron microscopy (TEM).

The amount of the particulate therapeutic agent used may vary from 0.01-5% by weight of the composition, usually from 0.01-1% by weight.

The mouthwash composition of the present invention further comprises a liquid medium. This medium may consist solely of water, or solely of an alcohol like ethanol, isopropanol, sorbitol, glycerol or mixtures thereof, or it may consist of an aqueous alcoholic medium.

The composition may furthermore optionally comprise conventional mouthwash ingredients such as flavours, sweetening agents, antibacterial agents, surface-active agents, colouring agents and so on.

The invention will further be illustrated by way of Example.

### Example 1

The following mouthwash compositions were prepared:

| | % by weight | | |
|---|---|---|---|
| | Control | A | B |
| ethanol | 6 | 6 | 6 |
| sodium fluoride | 0.055 | - | - |
| magnesium fluoride (aps 0.1 »m) | - | 0.04 | 0.04 |
| sorbitol (70% syrup) | 8 | 8 | 8 |
| glycerol | 4 | 4 | 4 |
| saccharin | 0.03 | 0.03 | 0.03 |
| flavour | 0.1 | 0.1 | 0.1 |
| nonionic surface-active agent | 0.1 | 0.1 | 0.1 |
| colouring agent | 0.03 | 0.03 | 0.03 |
| Polymer JR 400 | - | 0.01 | - |
| gelatin (Bloom strength 100) | - | - | 0.03 |
| demineralized water | q.s. | q.s. | q.s. |

In the manner as described in the afore-mentioned US Patent 4,837,007, Example 2, the free fluoride ion concentrations and the total fluoride concentrations were measured. The following results were obtained:

| Mouthwash | | Salivary Fluoride (ppm F) Time (mins) | | | |
|---|---|---|---|---|---|
| | | 60 | 90 | 120 | 180 |
| A | free F⁻ | 0.404 | 0.223 | 0.140 | 0.095 |
| A | total F | 3.728 | 1.362 | 0.598 | 0.249 |
| B | total F | 2.438 | 0.999 | 0.556 | 0.151 |
| Control(A) | free F⁻ | 0.233 | 0.123 | 0.076 | 0.056 |
| Control(B) | free F⁻ | 0.205 | 0.095 | 0.082 | 0.066 |

Compositions A and B had fully acceptable sensory properties and were stable on storage.

## Claims

1. An oral hygiene product in the form of a liquid mouthwash composition comprising from 0.01-5% by weight of the composition of a particulate therapeutic agent in a liquid medium which comprises a polymer, characterised in that the particulate therapeutic agent is suspended in the liquid medium with the aid of 0.0001-1% by weight of the composition of a polymer having an average molecular weight of 1,000,000 Daltons or less, selected from the group consisting of proteinaceous polymers, polysaccharide polymers, copolymers of vinylpyrrolidone with an acrylic monomer and mixtures thereof, said polymer having a net positive charge under the pH conditions of use of the mouthwash composition.

2. A product according to claim 1, characterised in that the polymer is gelatin.

3. A product according to claim 1, characterised in that the polymer is a quaternized cellulose derivative.

4. A product according to claim 1, characterised in that the polymer is a copolymer of vinylpyrrolidone with dimethylaminoethylmethacrylate.

5. A product according to claims 1-4, characterised in that the polymer has, under the pH conditions of use of the mouthwash composition, a cationic charge density of at least 0.0002, with the proviso that polysaccharide polymers derived from a guar gum have a cationic charge density of at least 0.0009.

6. A product according to claims 1-5, characterised in that the particulate therapeutic agent has a particle size within the range of 0.01-5 »m, and is selected from the group consisting of sparingly-soluble fluorides, sparingly-soluble zinc compounds, sparingly-soluble stannous salts, polymeric latices which release fluoride and mixtures thereof.

7. A product according to claim 6, characterised in that the particulate therapeutic agent is magnesium-fluoride

## Patentansprüche

1. Mundhygieneprodukt in Form einer flüssigen Mundspülung, umfassend in einer Menge von 0,01 bis 5 Gew.-% der Spülung ein teilchenförmiges therapeutisches Mittel in einem flüssigen Medium, das ein Polymer umfaßt, dadurch gekennzeichnet, daß das teilchenförmige therapeutische Mittel in dem flüssigen Medium mit Hilfe eines Polymers mit einem mittleren Molekulargewicht von 1 000 000 Dalton oder weniger in einer Menge von 0,0001 bis 1 Gew.-% der Spülung, ausgewählt aus der Gruppe, bestehend aus proteinischen Polymeren, Polysaccharidpolymeren, Copolymeren von Vinylpyrrolidon mit einem Acrylmonomer und Gemischen davon, suspendiert ist, wobei das Polymer eine insgesamt positive Ladung unter den bei der Verwendung der Mundspülung auftretenden pH-Bedingungen aufweist.

2. Produkt nach Anspruch 1, dadurch gekennzeichnet, daß das Polymer Gelatine ist.

3. Produkt nach Anspruch 1, dadurch gekennzeichnet, daß das Polymer ein quaternisiertes Cellulosederivat ist.

4. Produkt nach Anspruch 1, dadurch gekennzeichnet, daß das Polymer ein Copolymer von Vinylpyrrolidon mit Dimethylaminoethylmethacrylat ist.

5. Produkt nach Ansprüchen 1 bis 4, dadurch gekennzeichnet, daß das Polymer unter den zur Verwendung der Mundspülung vorherrschenden pH-Bedingungen eine kationische Ladungsdichte von mindestens 0,0002 aufweist, mit der Maßgabe, daß Polysaccharidpolymere, die von Guargummi abgeleitet sind, eine kationische Ladungsdichte von mindestens 0,0009 aufweisen.

6. Produkt nach Ansprüchen 1 bis 5, dadurch gekennzeichnet, daß das teilchenförmige therapeutische Mittel eine Teilchengröße im Bereich von 0,01 bis 5 »m aufweist und ausgewählt ist aus der Gruppe, bestehend aus schwerlöslichen Fluoriden, schwerlöslichen Zinkverbindungen, schwerlöslichen Zinn-(II)-verbindungen, polymeren Latizes, die Fluorid freisetzen, und Gemischen davon.

7. Produkt nach Anspruch 6, dadurch gekennzeichnet, daß das teilchenförmige therapeutische Mittel Magnesiumfluorid ist.

## Revendications

1. Produit d'hygiène buccale sous la forme d'une composition liquide de bain de bouche comprenant 0,01 % à 5 % en poids de la composition d'un agent thérapeutique en particules dans un milieu liquide qui contient un polymère, caractérisé en ce que l'agent thérapeutique en particules est en suspension dans le milieu liquide à l'aide de 0,0001 % à 1 % en poids de la composition d'un polymère ayant une masse moléculaire moyenne de 1 000 000 daltons ou moins, sélectionné dans le groupe comprenant des polymères protéïniques, des polymères de polysaccharides, des copolymères de vinylpyrrolidone avec un monomère acrylique et des mélanges de ceux-ci, ledit polymère ayant une charge totale positive dans les conditions de pH d'utilisation de la composition de bain de bouche.

2. Produit selon la revendication 1, caractérisé en ce que le polymère est de la gélatine.

3. Produit selon la revendication 1, caractérisé en ce que le polymère est un dérivé de cellulose quaternaire.

4. Produit selon la revendication 1, caractérisé en ce que le polymère est un copolymère de vinylpyrrolidone avec du diméthylaminoéthylméthacrylate.

5. Produit selon les revendications 1 à 4, caractérisé en ce que le polymère, dans les conditions de pH d'utilisation de la composition de bain de bouche, a une densité de charge cationique d'au moins 0,0002, à la condition que les polymères de polysaccharide dérivés d'une gomme de guar aient une densité de charge cationique d'au moins 0,0009.

6. Produit selon les revendications 1 à 5, caractérisé en ce que l'agent thérapeutique en particules a une taille de particule comprise entre 0,01 »m et 5 »m, et est sélectionné dans le groupe comprenant des fluorures difficilement solubles, des composés de zinc difficilement solubles, des sels stanneaux difficilement solubles, des réseaux polymères qui libèrent du fluorure et des mélanges de ceux-ci.

7. Produit selon la revendication 6, caractérisé en ce que l'agent thérapeutique en particules est du fluorure de magnésium.
